Europäisches Patentamt

European Patent Office

Office européen des brevets

(19)

(11) Publication number: **0 280 563**
**A2**

# (12) EUROPEAN PATENT APPLICATION

(21) Application number: **88301661.0**

(22) Date of filing: **26.02.88**

(51) Int. Cl.⁴: **C 07 K 15/00**
**A 61 K 37/02, C 12 P 21/00**

(30) Priority: **27.02.87 GB 8704646**

(43) Date of publication of application:
**31.08.88 Bulletin 88/35**

(84) Designated Contracting States:
**AT BE CH DE ES FR GB GR IT LI LU NL SE**

(71) Applicant: **THE WELLCOME FOUNDATION LIMITED**
**183-193 Euston Road**
**London NW1 2BP (GB)**

(72) Inventor: **Cozens, Peter John**
**The Wellcome Research Laboratories Langley Court**
**Beckenham Kent (GB)**

**Griffiths, Hazel Sylvia**
**The Wellcome Research Laboratories Langley Court**
**Beckenham Kent (GB)**

**Lewis, William Garnet**
**The Wellcome Research Laboratories Langley Court**
**Beckenham Kent (GB)**

**Rowe, Fiona Mary**
**The Wellcome Research Laboratories Langley Court**
**Beckenham Kent (GB)**

(74) Representative: **Stott, Michael John**
**The Wellcome Research Laboratories Group Patents &**
**Agreements Langley Court**
**Beckenham Kent BR3 3BS (GB)**

The microorganism(s) has (have) been deposited with American Type Culture Collection under number(s) CRL 1432.
Claims for the following Contracting States: ES + GR.

(54) **Improvements relating to cytotoxins.**

(57) A novel cytotoxin having anti-neoplastic activity and characterised by an apparent molecular weight of 47 ± 5 kDa, an apparent isoelectric point of at least 7.7, and an amino acid analysis. The cytotoxin may be obtained by culture and induction of a lymphoblastoid cell line.

**Description**

IMPROVEMENTS RELATING TO CYTOTOXINS

This invention relates to a novel cytotoxin, its isolation from the culture medium used for the incubation of lymphoblastoid cell lines, its formulation, and its use in human and veterinary medicine.

The cytotoxins are a group of proteins produced by cells of the immune system although different cytotoxins may be produced by different types of cells. Among other activities, these proteins are capable of killing tumour cells in culture and the two best characterised cytotoxins, tumour necrosis factors TNFα and TNFβ (also termed α-lymphotoxin), have been shown to possess anti-tumour activity in vivo. cDNAs encoding both TNFα and TNFβ have been cloned and expressed in E.coli (Pennica et al, Nature, 1984, 312, 724; Gray et al, Nature, 1984, 312, 721). Characterisation of other cytotoxins, such as natural killer cell cytotoxic factor (NKCF) and leukoregulin, has not been so extensive (Ortaldo J.R. et al, J. Immunol., 1986, 137, 2857-2863).

A novel cytotoxin, hereinafter referred to as basic lymphotoxin and abbreviated to bLT, has now been identified which has anti-neoplastic activity. In particular, it exhibits cytotoxic activity against a number of mammalian cell lines including especially the human colon adenocarcinoma cell line HT 29 (ATCC No. HTB 38) and the human nasal septum, quasi-diploid tumour cell line RPMI 2650 (ATCC No. CCL 30).

Accordingly, the present invention provides a cytotoxin having an apparent molecular weight by gel filtration of 47 ± 5 kDa, an apparent isoelectric point of at least 7.7, and the following amino acid analysis:

| Amino Acid | Number of Residues | Amino Acid | Number of Residues |
|------------|--------------------|------------|--------------------|
| Asx | 36.0 ± 4.3 | Pro | 24.5 ± 2.9 |
| Glx | 46.7 ± 5.6 | Tyr | 12.3 ± 1.5 |
| Ser | 41.0 ± 4.9 | Val | 22.7 ± 2.7 |
| Gly | 38.4 ± 4.6 | Met | 8.4 ± 1.0 |
| His | 13.6 ± 1.6 | Ile | 19.6 ± 2.4 |
| Arg | 19.6 ± 2.4 | Leu | 43.1 ± 5.2 |
| Thr | 21.4 ± 2.6 | Phe | 23.8 ± 2.9 |
| Ala | 33.2 ± 4.0 | Lys | 26.4 ± 3.2 |

In its native form, bLT is believed to have a quaternary structure composed of low molecular weight sub-units although this has not yet been established.

Preferably, bLT is provided in a substantially enriched form and especially in a form substantially free from materials with which it is normally associated in its native environment. In particular, the purity of bLT is greater than 90% and especially greater than 95% (w/w).

The bLT of the present invention has a combination of properties that is different from those of all previous cytotoxins that have been described. In addition to the above physico-chemical properties, bLT has the following biological properties that partcularly distinguish it from TNFα and/or TNFβ:

(i) It retains significantly more cytotoxic activity after incubation at 75°C than recombinant TNFα or recombinatn TNFβ;

(ii) Its cytotoxic activity is unaffected by treatment with neuraminidase whereas the cytotoxic activity of natural TNFβ is significantly diminished; and

(iii) Its cytotoxic activity on murine L929 cells (ATCC No. CCL 1)is not inhibited by lymphoblastoid interferon in contrast to natural TNFβ

The procedures by which the above physico-chemical and biological properties were established are described hereinafter.

The present invention also provides a process for producing bLT which comprises incubating a lymphoblastoid cell line in a culture medium, inducing the cell line with an inducing agent, and isolating bLT from the culture medium.

The process of the present invention extends to the use of any lymphoblastoid cell line but particularly to lymphoblastoid cell lines that are capable of producing interferon. (Strander, H, et al, J. Clin. Microbiol., 1975, 1, 116-124 and Christofinis, G.J. et al, J. Gen.Virol., 1981, 52, 169-171). Such cell lines are readily derived from the cells of patients with Burkitt's lymphoma, a tumour caused by Epstein-Barr virus. One particularly useful example of this cell line was derived from cells obtained from an African female child named Namalwa (Klein, G. et al, Int. J. Cancer, 1973, 12, 396-408). This cell line has been found to produce large amounts of interferon when suitably stimulated (Strander, H, et al, J. Clin. Micro., 1975 1, 116-117). A subculture of the cell line was obtained from Dr. I. Gresser (Villejuif, France) in January 1975. At that time the line was adapted to grow on medium RMPI 1640 (Moore, G.E., J. Amer. Med. Assoc., 1967, 199, 519-524) with 10% foetal calf serum. In these laboratories the cells have been adapted to grow on the same medium supplemented with 5-7% serum derived from 6-8 month old calves and they have been subcultured two or three times a week during an eighteen month period. A stock of these cells termed Namalwa/WRL has been laid down in a number of ampoules which are stored in liquid nitrogen. These cells were shown to be free from mycoplasma infection and samples have been deposited with the Americal Type Culture Collection (ATTC No. CRL 1432).

The procedure by which the lymphoblastoid cell line may be incubated and the culture medium for use therewith are generally known in the art. In a preferred aspect, the procedure is substantially the same as is used for interferon production. Thus, the cells are preferably grown in suspension in serum-containing culture medium at or about 37°C until a concentration of from 0.5 to 10 x 10⁶ cells/ml is obtained. At this stage, it may be desirable to recover the cells by, for example, centrifugation or filtration and to resuspend them in fresh culture medium. This affords the potential advantage of increasing cell density by using less medium (and hence increasing the yield of bLT) and/or of facilitating purification of bLT by employing a medium containing less or no serum for resuspension of the cells. On a production scale however these additional steps may not be justified.

An example of a serum-containing culture medium is RPMI 1640 supplemented with from 1 to 10% (v/v) of calf or horse serum. An example of a serum-free culture medium is single strength RPMI 1640 supplemented with the following:-

1mM glutamine
0.5% (v/v) tryptone
3.5 mg/ml glucose
1 mg/l insulin
0.14 mg/l putrescine
2 mg/l lecithin, and
2.2 mg/ml sodium bicarbonate.

Examples of preferred inducing agents include, viruses, especially Sendai virus, which may be used in association with an enhancer, such as a carboxylic acid or salt therof. The virus is preferably added to the cell culture to give a final concentration of from 5 to 200 HAU/ml, preferably 20 to 50 HAU/ml. After thorough mixing, the cell culture is incubated for a period of 12 to 48 hours at or about 37°C to allow induction to proceed to completion.

If, as is preferred, an enhancer is used, then it is usual to treat the cells with the enhancer once they have reached the required concentration of from 0.5 to 10 x 10⁶ cells/ml. At least when the process is carried out on a small scale, it is desirable to remove excess of the enhancer before induction by recovering the cells and resuspending them as described above. If a free carboxylic acid is used, then on addition to the culture medium, which will normally contain various inorganic or organic salts, the salts of the acid will usually be formed. If desired, it is possible to use the acid as the salt and add this to the medium. Examples of salts include sodium, potassium and ammonium salts.

It is especially preferred that induction of the cell line, particularly in association with the use of an enhancer, is carried out in substantially the same manner as is known for interferon production, for example as described in US patent 4216203.

Following incubation and induction of the cell line, bLT is present in the culture medium alongwith interferon and other products. bLT is preferably isolated from the culture medium by a process which includes the use of an anti-lymphoblastoid interferon antibody immunoaffinity column to remove the lymphoblastoid interferon and a chromatographic column to remove any cytotoxins have an acidic isoelectric point that may be present. In this way, bLT may be obtained in a substantially enriched form that is substantially free from lymphoblastoid and acidic cytotoxins.

The immunoaffinity column for the removal of lymphoblastoid interferon preferably comprises a cross-linked agarose gel, such as that sold under the trade name "Sepharose" (Pharmacia Ltd., Pharmacia House, Midsummer Boulevard, Central Milton Keynes, Bucks, England, MK9 3HP), to which are bound monoclonal or polyclonal anti-lymphoblastoid interferon antibodies. The culture medium is normally centrifuged or filtered first to remove the cells leaving a clarified liquid phase which is then applied to the immunoaffinity column. The effluent from the column, which includes bLT, is collected and may conveniently be concentrated at this stage using for example ultrafiltration.

The removal of the acidic cytotoxins using a chromatographic column may be accomplished by chromatofocusing or by ion-exchange chromatography of the optionally concentrated effluent from the immunoaffinity column. In either case, advantage is taken of the fact that the bLT of the present invention has an apparent isoelectric point of at least 7.7 whereas any acidic cytotoxin that may be present would have a significantly lower isoelectric point. This allows separation to be achieved by increasing the salt concentration or decreasing the pH of the eluant as appropriate to the type of exchanger. In the case of chromatofocusing, the column preferably comprises a cross-linked agarose gel with tertiary and quaternary amine functional groups. A specific example of such a gel is that sold under the trade name of "PBE 94" (Pharmacia Ltd). In the case of ion-exchange chromatography, the column preferably comprises a cross-linked agarose gel with diethylaminoethyl functional groups (an anion exchanger) or with carboxymethyl functional groups (a cation exchanger). Specific examples of such gels include those sold under the trade names of "DEAE-Sepharose CL-6B" and "CM-Sepharose CL-6B" respectively (Pharmacia Ltd.).

It may be advantageous to employ two ion-exchange columns to achieve fully effective separation of the bLT of the present invention from the acidic cytotoxins. Thus, one column, such as that comprising a cross-linked agarose gel with diethylaminoethyl functional groups, could be used to bind the acidic cytotoxins and allow the bLT to pass through and the second column, such as that comprising a cross-linked agarose gel with carboxymethyl functional groups, could be used to bind the bLT and allow the acidic cytotoxins to pass through.

The bLT-enriched solution may be purified further to achieve a level of purification greater than 90% (w/w) and especially greater than 95% (w/w) by methods known in the art including especially additional steps of column chromatography. If for example the culture medium in which the lymphoblastoid cells were incubated contained serum, then it would be desirable to pass the bLT-enriched solution down a polyclonal anti-serum immunoafinity column to remove any contaminating serum proteins. Examples of other columns which may preferably be employed include columns comprising a cross-linked agarose gel with Procion Red (HE-3B) and a monodisperse hydrophilic polymer with quaternary amine functional groups. Specific examples of such materials include those sold under the trade names "Red Sepharose CL-6B" and "Mono Q" respectively (Pharmacia Ltd).

The bLT of the present invention, or an immunogenic determinant thereof, may be used as an immunogen to raise antibodies by methods well known in the art. Polyclonal antibodies may be obtained by the immunisation of a laboratory animal with bLT, or an immunogenic deteminant thereof, and the recovery of the anti-sera. Monoclonal antibodies may be obtained by the immunisation of mice with bLT, or an immunogenic determinant thereof, the isolation of the sensitised spleen cells from the mice, and the fusion of such cells with myeloma cells to produce hybridoma cells, which are then cloned and cultured as required.

The polyclonal or monoclonal antibodies raised against bLT are of use in the purification of bLT by affinity chromatography and in the identification and characterisation of bLT.

As mentional previously, bLT has anti-neoplastic activity and hence is of use in the control and treatment of neoplastic diseases. It is of particular convenience in this respect to present bLT in a suitable form. Accordingly, the present invention provides a phamaceutical fomulation which comprises bLT and a pharmaceutically acceptable carrier.

The formulation is preferably an aqueous parenteral solution suitably adapted for administration by injection or infusion. It is optionally buffered at or about neutral pH and preferably contains saline or dextrose at a level sufficient to achieve substantial isotonicity with the blood serum of the patient. It may also contain a stabiliser, such as human serum albumin or an inert sugar. Conveniently, bLT may be presented in lyophilised form suitable for reconstition in water immediately prior to use.

The pharmaceutical formulation, whether wet or lyophilised, is preferably provided in sealed, sterile plastic or glass containers.

The present invention also provides a method for the treatment of neoplastic diseases in an animal which comprises administering to the animal a therapeutically effective amount of bLT. In the alternative, there is also provided bLT for use in human or veterinary medicine and in particular for use in the treatment of neoplastic diseases.

Optionally, bLT may be used alone or in combination with chemotherapeutic agents or other lymphokines including interferons, tumour necrosis factors, interleukins and colony stimulating factors.

The main routes of administration of bLT to the animal are intravascular, intratumour, intraperitoneal and intrapleural.

A therapeutically affective amount of bLT to treat an animal with a neoplasic disease will of course depend upon a number of factors including, for example, the age and weight of the animal, the precise disease requiring treatment and its severity, the route of administration and will utimately be at the discretion of the attendant physician. It is likely, however, that an effective amount will generally be in the range from 0.02 to 10 $\mu$g/kg, especially from 0.1 to 4 $\mu$g/kg, bodyweight of patient. Thus for a 70 kg adult human being, an effective amount will generally be from 1.4 to 700, especially from 7 to 280 $\mu$g.

The following examples are provided in illustration of the invention and should not be construed in any way as constituting a limitation thereof. The amount of protein bound to the various columns was monitored by the absorption of light at 280 nm ($A_{280}$).

Example 1 - Preparation of an Enriched Solution of bLT

Human lymphoblastoid cells of the Namalwa/WRL line were grown and induced as previously described (Finter N.B. et al, Cold Spring Harbour Symposia on Quantitative Biology, 1986, Vol. LI, 571-575). The resulting cell supernatant was passes through an immunoaffinity column of Sepharose (Pharmacia Ltd.) and anti-lymphoblastoid interferon polyclonal antibody constructed as previously described (Finter N.B., Fantes K.H. and Johnston M.D., Developments in Biological Standardisation, 1978, 38, 343-348; Fantes K.H., Burman C.J., Ball G.D., Johnston M.D. and Finter N.B., Biochemical Characterisation of Lymphokines, 1980, 343-351, Edited by A.L. de Weck, F. Kristensen and M. Lardy, New York and London, Academic Press) and run also as previously described (Secher D.S. and Burke D.C., 1980, Nature, 1980, 285, 446-450). This step removed substantially all of the lymphoblastoid interferon.

The bLT-containing column effluent (500 ml) was dialysed against 0.025M imidazole-HCL at pH 7.7 and applied to a 1cm x 30cm column of PBE 94 (Pharmacia Ltd.) previously equilibrated with the same buffer. The loading flow rate was 11 ml/hr and 11 ml fractions were collected while the sample was being applied. The column was then washed with 100 ml of the original imidazole-HCl buffer before being eluted with a pH gradient formed from 500 ml of eight times diluted Polybuffer 74 (Pharmacia Ltd.) at pH 4.0. During elution the fraction size was changed to 3 ml. At least 80% of the material ($A_{280}$) bound to the column and could hence be separated from bLT by this procedure. Typically, the material loaded onto the column had a protein concentration of approximately 2 mg/ml and 56 U/ml of cytotoxic activity while the bLT-containing column effluent was approximately 0.33 mg/ml containing 40 U/ml of cytotoxic activity.

A cytotoxic activity, also present in the starting material, bound to the column and was eluted over the pH range 5.3 - 6.3, with a peak at pH 5.7.

The assay for cytotoxic activity utilised LM cells (ATCC No. CCL 1.2) and neutral red dye and was carried out as follows:-

LM cells were seeded in microtitre wells at $5 \times 10^4$ cells/ml (200 µl per well) in RPMI 1640 medium containing 1% foetal calf serum. Dilutions of the assay material were made in RPMI 1640 medium containing 1% foetal calf serum. 50 µl samples of each dilution were added to duplicate wells, immediately after the cells had been added to the wells. The microtitre plates were incubated at 37°C in 5% carbon dioxide. After 64-72 hours incubation, the cells were stained for 1.5 hours in 0.036% neutral red, 0.9% NaCl and 0.015N HCl (50 µl/well). The staining solution was removed by aspiration and the cells washed once with 0.9% NaCl solution. The cells were lysed with 200 µl/well of lysis solution (50% (v/v) 0.9% NaCl/ethanol) for 30 minutes at room temperature and the absorbance at 540 nm was determined.

## Example 2 - Alternative Preparation of an Enriched Solution of bLT

Approximately 40 litres of the effluent from the anti-lymphoblastoid interferon polyclonal antibody immunoaffinity column described in Example 1 were concentrated and simultaneously dialysed against 0.01 M imidazole-HCl at pH 7.7 using an Asahi artifical kidney (AM300; Kimal Scientific Products Ltd., Uxbridge, Middlesex, England). The final volume was approximately 2.5 litres.

Approximately 2 litres of this concentrated material was applied to a 2.5 cm x 18 cm column of CM-Sepharose (Pharmacia Ltd.) previously equilibrated in 0.01M imidazole-HCl at pH 7.7. Flow rate through the column was 50 ml/hr and three bulk fractions of effluent were collected. The column was washed with 250 ml of the same imidazole-HCl buffer and the effluent was collected as 10 x 25 ml fractions. The column was then eluted with a linear gradient of increasing ionic strength formed from 1 litre of the initial imidazole-HCl buffer and 1 litre of the same buffer containing 1M sodium chloride. During elution, 10 ml fractions wre collected.

The amount of protein present in both the effluent and eluate solutions was such that the elution profile determined by $A_{280}$ was off-scale and so no estimate of amount of material bound to the column could be made.

Various fractions across the elution profile were assayed for cytotoxic activity using LM cells and neutral red dye as described in Example 1. Activity was detected in two areas, at 0.08 to 0.11M NaCl and at 0.28 to 0.33M NaCl. These could represent two different forms of bLT.

## Example 3 - Preparation of a Purified Solution of bLT

Human lymphoblastoid cells of the Namalwa/WRL line were grown in RPMI 1640 medium supplemented with 7% (v/v) adult bovine serum, 0.5% (w/v) tryptone and 3 mg/ml glucose. When the cell density reached 2.5-3.0 x $10^6$ cells/ml, the culture was diluted with RPMI 1640 supplemented with 7% adult bovine serum to a density of 1.0-1.5 x $10^6$ cells/ml. 1M soldium butyrate was added to give a final concentration of 2 mM. The culture was held at 37°C with agitation for 2 days. After this time the cells were centrifuged at 800 rpm for 10 minutes in a Mistral 6 litre centrifuge (M.S.E., Manor Royal, Crawley, W. Sussex, England). The sedimented cells were washed and resupeneded in serum-free RPMI 1640 supplemented with 1mM glutamine; 0.5% (w/v) tryptone; 3.5 mg/ml glucose; 1 mg/litre insulin; 0.14 mg/litre putrescine; 2 mg/litre lecithin; and 2.2 mg/ml sodium bicarbonate. Allantoic fluid containing Sendai virus was added at 1% (v/v). Following incubation at 37°C for 24 hours, the supernatant was harvested by centrifugation at 1500 rpm for 30 minutes in a Mistral 6 litre centrifuge followed by filtration through a 0.22 µm filter.

The supernatant was passed over an anti-lymphoblastoid interferon polyclonal antibody immunoaffinity column constructed and used as described in Example 1. 10 litres of effluent from the column were concentrated to 0.5 litres and simultaneously diafiltrated against a solution of 25 mM imidazole-HCl at pH 7.4 using an Amicon spiral-wound ultrafiltration cartridge with a YM10 membrane (Amicon Ltd.). The concentrated solution was further dialysed against a solution of 25 mM imidazole-HCl at pH 7.4 at 4°C overnight.

The concentrated bLT-containing solution was then applied to two ion-exchange columns run in series: a 2.5 cm x 5 cm column of DEAE-Sepharose Cl-6B (Pharmacia Ltd.) followed by a 2.5 cm x 2.5 cm column of CM-Sepharose CL-6B (Pharmacia Ltd.). Both columns were pre-equilibrated with a solution of 25 mM imidazole-HCl at pH 7.4 at 4°C. The effluent from the column of DEAE-Sepharose CL-6B was applied directly to the CM-Sepharose column. Using stepwise increases in NaCl concentration (50 mM, 100 mM and 250 mM), the Cm-Sepharose CL-6B column was eluted at a flow rate of 1 ml per minute until the $A_{280}$ returned to the baseline. The major bLT-containing peak was eluted in the presence of 100 mM NaCl in a volume of 30 ml.

The bLT-containing eluate was applied directly to a 2 cm x 1.6 cm column of Red Sepharose CL-6B (Pharmacia Ltd). The bLT bound to the column and was eluted with 50 mM diethanolamine-HCl - 0.1 M arginine at pH 8.4 at a flow-rate of 0.5 ml per minute until the $A_{280}$ returned to the baseline. This required a volume of 15 ml. A 5-fold purification was achieved with this step.

The bLT-containing eluate from the Red Sepharose column was diluted (1:4) with 50mM diethanolamine-HCl at pH 9.4 and applied to a column of Mono Q (Pharmacia Ltd.) at a flow-rate of 1 ml per minute. All cytotoxic activity was recovered in the non-bound fraction.

The specific activities of samples of bLT obtained during the purification procedure were determined using the assay based on LM cells and neutral red dye as described in Example 1. The results are tabulated below:-

| Sample | Total Protein Concentration (mg/ml)* | bLT (U/ml) | Specific Activity (U/mg) |
|---|---|---|---|
| 1. Post Anti-lymphoblastoid interferon immunoaffinity column | 0.12 | 150 | $1.25 \times 10^3$ |
| 2. Post CM-Sepharose column | 0.085 | 800 | $9.4 \times 10^4$ |
| 3. Post Red-Sepharose column | 0.012 | 8100 | $7.2 \times 10^5$ |
| 4. Post Mono Q column | 0.008 | 9750 | $1.2 \times 10^6$ |

\* Determined using a Bio-Rad protein assay kit (Bio-Rad Laboratories, Richmond, California, U.S.A.).

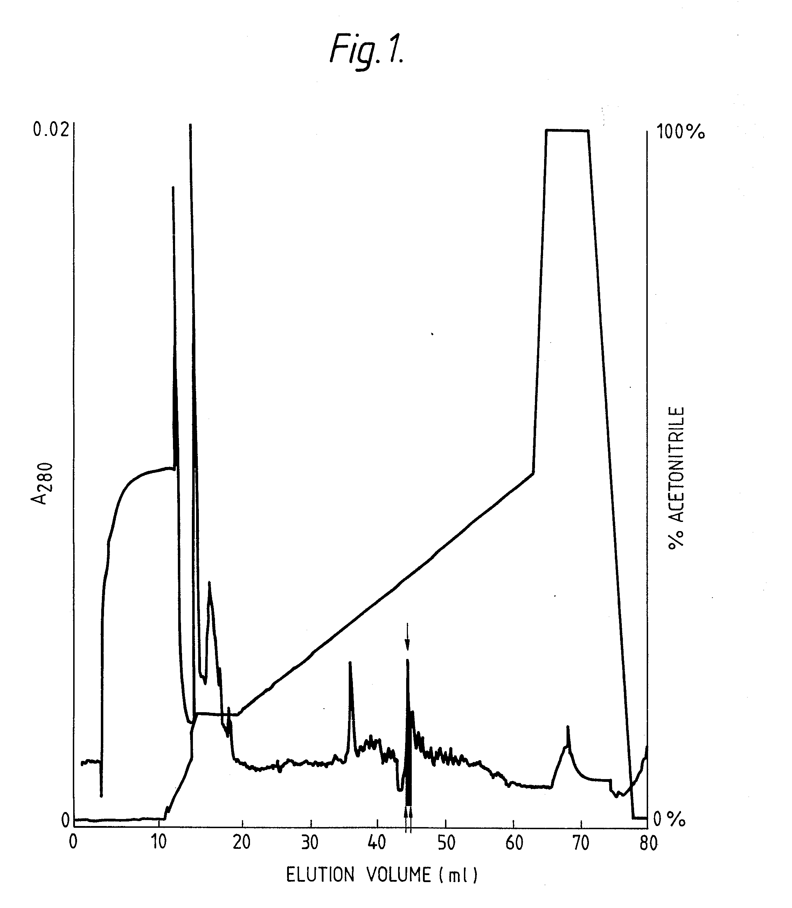

For amino acid analysis only, the bLT was subjected to further purification involving reversed phase chromatography on a Pharmacia FPLC system with a column of Pro RPC (Pharmacia Ltd.). The sample was applied directly from the Mono Q column and eluted with a gradient of 0-15-40% of acetonitrile in 0.1% trifluoroacetic acid. The flow-rate was 0.7 ml per minute. The first 15% increase in acetonitrile concentration took place at a rate of 5% per minute. It was then held at 15% for five minutes and thereafter the gradient change was 1% per minute. The bLT was detected by ELISA in a peak of the $A_{280}$ trace, of which a copy is set forth in Figure 1, the peak eluting at 32-35% acetonitrile.

Example 4 - Production of Monoclonal Antibodies against bLT

The procedure for the production of hybridomas was largely as described by Kohler, G. et al, Nature, 1975, 256, 495-497 except as indicated below:-

(i) Two BALB/c mice were inoculated intraperitoneally and subcutaneously at multiple sites with an estimated 750 ng/mouse of bLT in Freund's complete adjuvant, the bLT having been obtained by the procedure of Example 2 and additionally applied to an anti-bovine serum immunoaffinity column.

(ii) After one month each mouse was boosted with 125 ng bLT in Freund's incomplete adjuvant using the routes described above, the bLT having been obtained as in (i) and further purified of gel filtration on a cross-linked agarose matrix sold under the trade name "Sepharose 12" (Pharmacia Ltd.).

(iii) After a further three weeks each mouse was boosted subcutaneously and intravenously with 1.75 µg of bLT in Alhydrogel (2% aluminium hydroxide in water), the bLT having been obtained from the column of CM-Sepharose as described in Example 3 and having been further purified by gel filtration.

(iv) The final boost, one month later, used 5.75 µg bLT/mouse which was given intravenously, the bLT having been obtained and purified as in (iii). After three days the mice were sacrificed and the spleen cells used for fusion with P3-NSI/I-Ag4-I myeloma cells (J. Mol. Biol., 1974, 90, 691).

The resulting hydridomas were cloned and cultured in accordance with procedures known in the art. The cloned hybridomas were screened using an immunoradiometric or immunodot blot assay in conjunction with an assay that detected depletion of activity by immunoprecipitation. Individual clones were thus selected for their ability to secrete the desired antibody.

Example 5 - Physico-Chemical Properties of bLT

6

## A. Molecular Weight

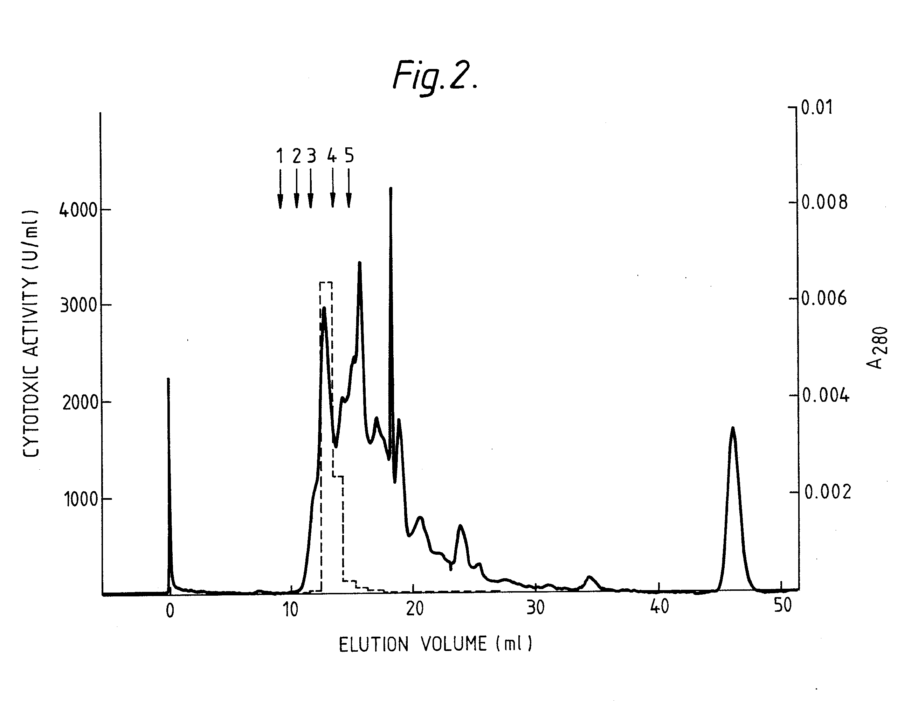

The apparent molecular weight of bLT was determined by gel filtration. The bLT (200 µl) was obtained from the column of CM-Sepharose as described in Example 3 and concentrated five times on an Amicon stirred cell with a YM10 membrane (Amicon Ltd.). The concentrated sample was applied to a gel filtration column of Superose 12 HR 10/30 (Pharmacia Ltd.) and eluted with phosphate buffered saline at pH 7.2 at a rate of 0.5 ml/min. The column eluate was assayed for cytotoxic activity using LM cells and neutral red dye as described in Example 1. The results are depicted in Figure 2 in which the dotted line corresponds to cytotoxic activity and in which the numbered arrows correspond to the elution position of proteins of known molecular weight. The identity of the known proteins is as follows:-

1. Apoferritin
2. β-Amylase
3. Bovine serum albumin
4. Carbonic anhydrase
5. Cytochrome C

From Figure 2 an apparent molecular weight for bLT of 47 ± 5 kDa was thus obtained.

## B. Isoelectric Point

In Example 1 the bLT contained in the effluent from the anti-lymphoblastoid interferon immunoaffinity column did not bind to the chromatofocusing column of PBE 94 (Pharmacia Ltd.) under the conditions described. Its chromatographic behaviour thus allows the conclusion that the apparent isoelectric point of bLT is greater than 7.7.

## C. Aminoacid Analysis

bLT obtained from the column of Pro RPC as described in Example 3 was hydrolysed with 6N HCl and subjected to amino acid analysis using a Waters "Pico-Tag" system in accordance with methods known in the art. The calculated amino acid composition was based on a molecular weight of 47 kDa.

## Example 6 - Biological Properties of bLT

### A. Cytotoxic Activity of bLT, TNFα and TNFβ

The target mammalian cell lines were all subjected to identical procedures to assess them for sensitivity to cytotoxic activity. The procedures were as follows:-

The cell lines were grown in RPMI 1640 medium containing 5-10% foetal calf serum. At the end of the exponential growth phase, the medium was replaced with RPMI 1640 containing 1% foetal calf serum. 200 µl volumes were taken, each containing $10^4$ cells, and distributed into 0.3 ml wells of microtitre trays (Costar).

Replicate $\log_2$ serial dilutions of bLT, obtained from the Mono Q column according to the procedure of Example 3 and having a concentration of 3250 U as determined on LM cells, were made using RPMI 1640 containing 1% foetal calf serum. The dilutions were added (50 µl/well) immediately to the target cells in two replicate rows containing identical dilutions. The target cells were then incubated in a humidified incubator at 37°C in 5% carbon dioxide for 48 hours. At the end of this period, 4 µCi [methyl-$^3$H] thymidine (Amersham International, England; 52 Ci/mM) in 40 µl of the same medium were added to each well. The target cells were allowed to label for 18 hrs at 37°C in 5% carbon dioxide. The medium was then removed from adherent cell types (LM, L929, RPMI 2650) and replaced with 200 µl 0.05% ethylene diaminetetraacetic acid and incubated for 10 minutes at room temperature. All cells were washed and harvested onto glass fibre filter discs (Whatman GF/B) using a cell harvester (M-48R, Brandel, USA) with phosphate buffered saline at pH 7.2 as the wash solution. Each filter disc was placed in a glass vial with 5 ml scintillant sold under the trade name "Cocktail W" (British Drug Houses, Poole, Dorset, England) and counted using a liquid scintillation counter. Units of bLT required to kill 50% of each target cell were determined in comparison to LM cells by probit analysis (J. Gen. Virol, 1972, 14, 49).

The various cell lines which were used and the results obtained are as tabulated below:-

|  | Number of LM Units of bLT |
| Target Cell | to cause 50% Cytotoxicity |
| --- | --- |
| LM | 1 U/ml |
| RPMI 2650 | 20 U/ml |
| L929 | 20 U/ml |
| MOLT-4 | 25 U/ml |
| K562 | 800 U/ml |
| RAJI | >1000 U/ml |
| DAUDI | >1000 U/ml |
| HL60 | >1000 U/ml |

| Cell Line | ATCC No. | Nature |
| --- | --- | --- |
| LM | CCL 1.2 | Murine tumourigenic fibroblast |
| RPMI 2650 | CCL 30 | Human nasal septum; quasi-diploid tumour |
| L929 | CCL 1 | Murine tumourigenic fibroblast |
| MOLT-4 | CRL 1582 | Human peripheral blood; acute lymphoblastic leukemia |
| K562 | CCL 243 | Human chronic myelogenous leukemia |
| RAJI | CCL 86 | Human Burkitt's lymphoma |
| DAUDI | CCL 213 | Human Burkitt's lymphoma |
| HL 60 | CCL 240 | Human peripheral blood promyelocytic leukemia |

Using the human colon adenocarcinoma cell line HT 29 (ATCC No. HTB 38), the cytotoxic activities of bLT, natural TNFα and natural TNFβ were determined using a tritium release assay as described below. The bLT was obtained from the anti-lymphoblastoid interferon immunoaffinity column as described in Example 1. Natural TNFα was used in the form of a supernatant from lipopolysaccharide stimulated J774.1 cells (Nature, 1975, 257, 393-394). Natural TNFβ was also used in the form of a supernatant prepared according to the following procedure:-

RPMI 1788 cells (ATCC No. CCL 156) were seeded ($6 \times 10^6$ cells/ml) in serum free RPMI 1640 medium in the presence of 2 ng/ml phorbol myristate acetate. The cells were incubated for 3 hours at 37°C in 5% carbon dioxide. Phytohaemagglutinin was added to a final concentration of 5 µg/ml and the culture incubated for 48 hours. the cells were removed by centrifugation, the supernatant checked for TNFβ activity on L929 cells, and then concentrated on an Amicon filtration cell with a YM 10 membrane (Amicon Ltd.).

The tritium release assay was carried out as follows:-

Target cells were labelled by growing in Dulbecco's modification of Eagle's medium supplemented with 25 mM HEPES, 5% heat inactivated foetal calf serum and 5 µCi/ml methyl [³H]-thymidine (2 Ci/mmol). Cell monolayers were washed twice in phosphate buffered saline without calcium or magnesium to remove surplus labelling agent, and then removed fromthe plastic trays using 0.05% ethylenediamine tetraacetic acid. The labelled target cells were dispersed in Dulbecco's modification of Eagle's medium and 200 µl volumes (each containing $10^4$ cells) were distributed into 0.3 ml wells of microtitre trays (Costar), and then incubated in a humidified incubator at 37°C in 5% carbon dioxide for 18 hours. The medium was aspirated off and replaced with sample dilutions (250 µl/well) in two replicate rows containing identical $\log_3$ serial dilutions. The plates were then reincubated for 24 hours. Cycloheximide was added to a final concentration of 1 µg/ml and the plates reincubated for a further 24 hours. 200 µl volumes of supernatant from the wells were aspirated into the scintillant, "Cocktail W", and counted using a liquid scintillation counter.

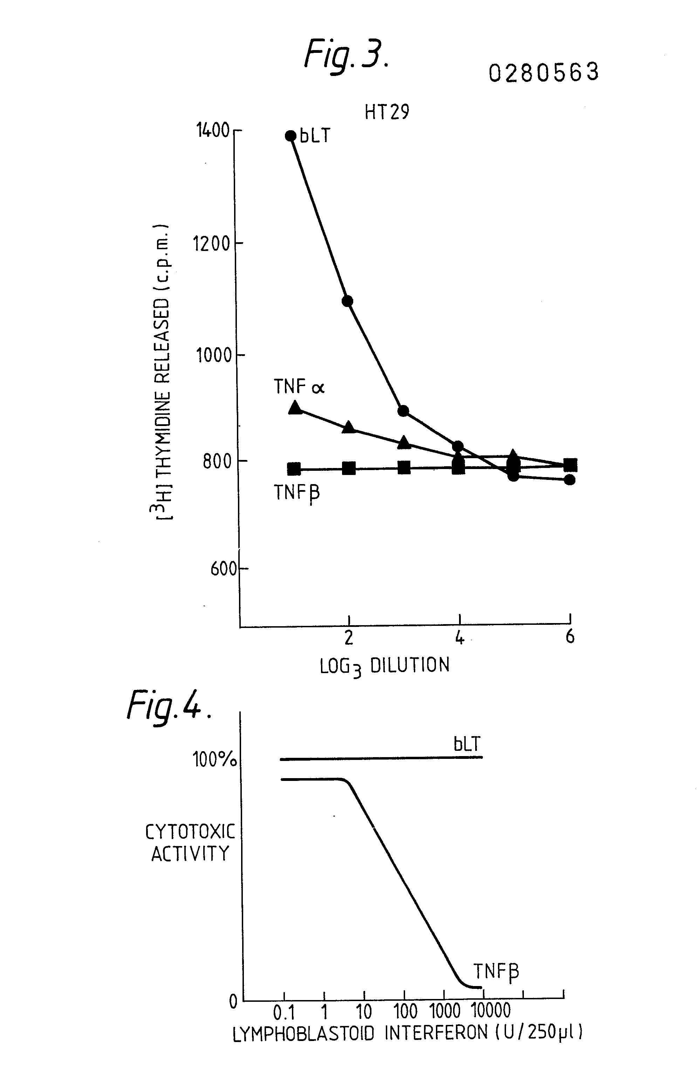

The results are represented graphically in Figure 3.

B. Effect of temperature on Cytotoxic Activity of bLT, rTNFα and rTNFβ

Samples (500 U/ml) of purified bLT, obtained from the Mono Q column according to the procedure of Example 3, were incubated at various temperatures for 30 minutes. They were then assayed using the LM cell line and neutral red dye as described in Example I.

Similarly, samples of recombinant TNFα (Genzyme Fine Chemicals Ltd., Suffolk, England) and recombinant TNFβ (NIBSC, South Mimms, Potters Bar, Hertfordshire, England) were also incubated at a number of the same temperatures and assayed in the same way to afford a basis for comparison. The results are tabulated below:-

| Temperature | % Cytotoxic Activity Retained | | |
|---|---|---|---|
| | bLT | rTNFα | rTNFβ |
| 20°C | 100 | 100 | 100 |
| 42°C | 100 | ND | ND |
| 56°C | 100 | ND | ND |
| 65°C | 100 | ND | ND |
| 75°C | 81 | 9 | 63 |
| 80°C | 6.5 | 5 | 6 |

ND — Not determined

The results for rTNFα and rTNFβ were obtained from one experiment. The results for bLT at 75°C and 80°C were the mean of three and two experiments respectively. Otherwise the results for bLT were also obtained from one experiment.

The results at 75°C indicate a statistically meaningful difference in temperature stability between bLT and rTNFα and rTNFβ.

C. Effect of Neuraminidase on Cytotoxic Activity of bLT and TNFβ

Samples of bLT, obtained form the Mono-Q column according to the procedure of Example 3, were treated with neuraminidase from vibrio cholerae (BDH Chemicals Ltd., Poole, Dorset, England) as follows:-

5 µl Neuraminidase (500 U/ml) was added to each 25 µl sample (2500 U/ml) together with 50 µl 0.1M sodium acetate buffer at pH 5.1, and incubated for 60 minutes at 37°C. 5 µl Sialic acid (0.2 M) was added to each sample to inactivate the enzyme. The volume was adjusted to 0.3 ml with RPMI 1640 medium containing 1% foetal calf serum, and then assayed for activity using the LM cell line and neutral red dye as described in Example I. As a control for specificity of the enzyme, samples were also incubated with neuraminidase, sodium acetate buffer and 0.2 M sialic acid before assay.

The results indicate that the cytotoxic activity of bLT is fully retained following treatment with neuraminidase. This is in contrast to published observations with TNFβ from RPMI 1788 cells which indicate that the cytotoxic activity of TNFβ is diminished 49 and 92% by 0.2 and 0.4 units of neuraminidase (Cancer Research, 1985, 45, 851-862).

D. Effect of Chymotrypsin on Cytotoxic Activity of bLT, rTNFα nd rTNFβ

Samples of bLT, obtained from the Mono Q column according to the procedure of Example 3, recombinant TNFα and recombinant TNFβ, obtained as indicated above, were incubated with chymotrypsin to assess the susceptibility of each protein to digestion by this enzyme. The procedure was as follows:-

300 µl of each sample (500 U/ml as assayed using the LM cell line and neutral red dye as described in Example I) was incubated with 6 units of chymotrypsin (Type I-S; Sigma Chemicals Ltd., Poole, Dorset, England) for 2 hours at 37°C. After this time Soybean trypsin inhibitor (Sigma Chemicals Ltd.,) was added to each sample to inhibit the reaction. The samples were then assayed using the LM cell line and neutral red dye as described in Example I.

The results show that bLT and rTNFβ are largely unaffected by chymotrypsin whereas the cytotoxic activity of rTNFα is reduced to below detectable levels. (The limit of detection with the assay was 20 U/ml).

E. Effect of Lymphoblastoid Interferon on Cytotoxic Activity of bLT and TNFβ.

The cytotoxic activities of bLT, obtained from the anti-lymphoblastoid interferon immunoaffinity column according to the procedures of both Examples 1 and 3, and natural TNFβ, obtained according to the procedure described in Example 6A, were determined both in the absence and presence of 0.1 to 10,000 U per well of lymphoblastoid interferon ("Wellferon"). The cell line used was L929 and the assay was the tritium release assay described in Example 6A. The results are represented graphically in Figure 4.

No inhibition of the cytotoxic activity of bLT was observed whereas the cytotoxic activity of TNFβ was inhibited in the presence of concentrations of lymphoblastoid interferon greater than 10 U per well. The result with TNFβ is in agreement with published observations (Williams, Lymphokine Research, 1984, 3, 113).

F. Assay of bLT-Containing Column Effluent for Interleukin-1 and γ-Interferon Activities.

(i) Using the effluent from the anti-lymphoblastoid interferon immunoaffinity column described in example 1, an assay for interleukin-1 activity was carried out using the C3H/He comitogenesis assay (Eur. J. Immunol., 1986, 16, 370-375). No such activity was detected.

(ii) Using the effluent from the PBE 94 column described in Example 1, an assay for γ-interferon activity was carried out using an immunoradiometric assay (Sucrosep Kit, Boots-Celltech Diagnostics Ltd., England). The level of activity was shown to be about 1.5 units/ml.

## Claims

1. A cytotoxin having an apparent molecular weight by gel filtration of 47 ± 5 kDa, an apparent isoelectric point of at least 7.7, and the following amino acid analysis:

| Amino Acid | Number of Residues | Amino Acid | Number of Residues |
|------------|--------------------|------------|--------------------|
| Asx | 36.0 ± 4.3 | Pro | 24.5 ± 2.9 |
| Glx | 46.7 ± 5.6 | Tyr | 12.3 ± 1.5 |
| Ser | 41.0 ± 4.9 | Val | 22.7 ± 2.7 |
| Gly | 38.4 ± 4.6 | Met | 8.4 ± 1.0 |
| His | 13.6 ± 1.6 | Ile | 19.6 ± 2.4 |
| Arg | 19.6 ± 2.4 | Leu | 43.1 ± 5.2 |
| Thr | 21.4 ± 2.6 | Phe | 23.8 ± 2.9 |
| Ala | 33.2 ± 4.0 | Lys | 26.4 ± 3.2 |

2. A cytotoxin according to claim 1 having a purity which is greater than 90% (w/w).

3. A cytotoxin according to claim 2 having a purity which is greater than 95% (w/w).

4. A pharmaceutical formulation which comprises a cytotoxin according to any one of the preceding claims and a pharmaceutically acceptable carrier.

5. A pharmaceutical formulation according to claim 4 which is adapted for administration by injection or infusion.

6. A cytotoxin according to any one of claims 1 to 3 for use in human and veterinary medicine.

7. A cytotoxin according to any one of claims 1 to 3 for use in the treatment of neoplastic diseases.

8. Polyclonal or monoclonal antibodies against a cytotoxin according to any one of claims 1 to 3.

9. A process for producing a cytotoxin to any one of claims 1 to 3 which comprises incubating a lymphoblastoid cell line in a culture medium, inducing the cell line with an inducing agent, and isolating the cytotoxin from the culture medium.

10. A process according to claim 9 wherein the isolation of the cytotoxin is carried out using an anti-lymphoblastoid interferon antibody immunoaffinity column to remove the lymphoblastoid interferon and a chromatographic column to remove cytotoxins having an acidic isoelectric point.

Claims for the following contracting States: ES, GR

1. A process for producing a cytotoxin having an apparent molecular weight by gel filtration of 47 ± 5 kDa, an apparent isoelectric point of at least 7.7, and the following amino acid analysis:

| Amino Acid | Number of Residues | Amino Acid | Number of Residues |
|---|---|---|---|
| Asx | 36.0 ± 4.3 | Pro | 24.5 ± 2.9 |
| Glx | 46.7 ± 5.6 | Tyr | 12.3 ± 1.5 |
| Ser | 41.0 ± 4.9 | Val | 22.7 ± 2.7 |
| Gly | 38.4 ± 4.6 | Met | 8.4 ± 1.0 |
| His | 13.6 ± 1.6 | Ile | 19.6 ± 2.4 |
| Arg | 19.6 ± 2.4 | Leu | 43.1 ± 5.2 |
| Thr | 21.4 ± 2.6 | Phe | 23.8 ± 2.9 |
| Ala | 33.2 ± 4.0 | Lys | 26.4 ± 3.2 |

which comprises incubating a lymphoblastoid cell line in a culture medium, inducing the cell line with an inducing agent, and isolating the cytotoxin from the culture medium.

2. A process according to claim 1, wherein the isolation of the cytotoxin is carried out using an anti-lymphoblastoid interferon antibody immunoaffinity column to remove the lymphoblastoid interferon and a chromatographic column to remove cytotoxins having an acidic isoelectric point.

3. A process according to either of the preceding claims, wherein the cytotoxin is obtained in a purity which is greater than 90% (w/w).

4. A process according to claim 3, wherein the purity is greater than 95% (w/w).

0280563

Fig.1.

Fig.2.

Fig.3.

0280563

HT29

Fig.4.